(19) Europäisches Patentamt
European Patent Office
Office européen des brevets

(11) **EP 4 318 038 A1**

(12) **EUROPEAN PATENT APPLICATION**

(43) Date of publication:
**07.02.2024 Bulletin 2024/06**

(21) Application number: **22315178.8**

(22) Date of filing: **04.08.2022**

(51) International Patent Classification (IPC):
**G01S 7/52** *(2006.01)* **A61B 8/08** *(2006.01)*

(52) Cooperative Patent Classification (CPC):
**G01S 7/52036; A61B 8/5207;** A61B 8/469;
A61B 8/5223; A61B 8/5269

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **SUPERSONIC IMAGINE**
**13290 Aix en Provence (FR)**

(72) Inventors:
• **Lambert, William**
**94200 Ivry sur Seine (FR)**
• **Ilyina, Natalia**
**13290 Aix-en-Provence (FR)**
• **Zhang, Bo**
**13109 Simiane-Collongue (FR)**

(74) Representative: **Paustian & Partner Patentanwälte mbB**
**Oberanger 32**
**80331 München (DE)**

(54) **METHOD AND SYSTEM FOR EVALUATING ULTRASOUND DATA FOR THE PURPOSE OF ULTRASOUND ATTENUATION ESTIMATION IN A MEDIUM**

(57)    The invention relates to a method of evaluating ultrasound data for the purpose of ultrasound attenuation estimation in a medium, the medium being associated with ultrasound spatio-temporal signal data received from the medium in response to at least one ultrasound plane and/or diverging wave emitted into the medium, wherein the method comprises:

beamforming a first subset of ultrasound spatio-temporal signal data to obtain a second subset of beamformed ultrasound data,
determining a focusing quality criterion from the first subset, and
evaluating the second subset as a function of the focusing quality criterion of the first subset.

Fig. 3a

EP 4 318 038 A1

**Description**

BACKGROUND

It is known to use a plurality of transducer elements or transceivers (for example arranged as an array) for communication, imaging or scanning purposes, for example in the field of medical imaging, radar, sonar, seismology, wireless communications, radio astronomy, acoustics, and biomedicine. One example comprises ultrasound imaging.

The aim of ultrasound imaging is to estimate the medium reflectivity. In a conventional ultrasound imaging method, an ultrasound transducer device (also referred to as an ultrasound probe) with one or a set of ultrasound transducer elements may be used. In the method, one or multiple transducers are used to transmit one or successively several ultrasound beam into a medium, corresponding to a transmission operation. Then, in a reception operation a set of backscattered echo signals are received from the medium by the same or another set of transducer element(s). In particular, each of the transducer elements converts a received echo signal into for example an electrical signal. The signal may further be processed by the ultrasound system. For example, they may be amplified, filtered digitalized and/or a signal conditioning operation may be carried out. The transducer elements may be arranged as a transducer array.

Conventionally, said signals are then transmitted to an image processing system. The received signals may be processed to generate image data of the scanned medium, for example using a beamforming method. Generally, beamforming may be understood as a signal processing technique conventionally used in sensor arrays for directional signal transmission or reception. This process is used to generate beamformed data. In other words: beamforming may be understood as a signal processing technique that is achieved by combining elements in an antenna array (for example an ultrasound transducer) in such a way that the combined signals form constructive interferences.

However, a phenomenon in ultrasound imaging, which desirably has to be considered, is ultrasound attenuation within an examined medium. Ultrasound attenuation directly affects the response to transmitted ultrasound waves. Attenuation thereby constitutes a subtle frequency and depth dependent phenomenon. It is thus desirable to compensate any effects of attenuation on the resulting computed image, as it is conventionally done by for example time-gain compensation to account for tissue attenuation.

On the other hand, a reliable estimation of ultrasound attenuation may be used for other ultrasound diagnosis purposes. For example, a global attenuation parameter estimated for the examined medium, for example a human liver may serve for determining its fat content. Moreover, a local attenuation distribution, for example processed to compute an image, may serve for detecting cancers, for example in a human breast or fibers in muscle.

SUMMARY OF THE DISCLOSURE

The system and method described herein are related to technologies for evaluating ultrasound data for the purpose of ultrasound attenuation estimation in a medium, which advantageously is more precise (i.e. accurate) and/or more reliable. Moreover the method of the present disclosure is desirably computationally less expensive and thus requires less processing power and being compliant with real time constraints.

Therefore, a method of evaluating ultrasound data is provided, desirably for the purpose of ultrasound attenuation estimation in a medium. The medium is associated with ultrasound spatio-temporal signal data received from the medium in response to at least one ultrasound plane and/or diverging wave emitted into the medium. The method comprises: beamforming a first subset of ultrasound spatio-temporal signal data to obtain a second subset of beamformed ultrasound data, determining a focusing quality criterion from the first subset, and evaluating the second subset as a function of the focusing quality criterion of the first subset.

By providing such a method, it becomes possible to evaluate the ultrasound data (i.e. the second set) based on a focusing quality criterion. Said focusing quality criterion may comprise valuable information to qualify the second subset for the purpose of attenuation estimation. The focusing quality criterion may for example also be referred to as a focusing quality value. In particular, the focusing quality criterion may serve to identify homogeneous areas in the medium, but may also provide further relevant information, as for example a level of noise of the ultrasound spatio-temporal signal data used to form the first subset.

Furthermore, since beamforming the first subset and determining the focusing quality criterion may be carried out simultaneously, the method may be faster and may provide an evaluation result in real-time. Moreover, since the selection of a first subset (as described in detail below) may be used for both operations (beamforming and determining), the method may be computationally less expensive.

Moreover, since desirably plane and/or diverging waves may be used to obtain the ultrasound spatio-temporal signal data, focusing quality criteria of different regions of the medium may be calibrated, i.e. comparable with each other. The provision of ultrasound spatio-temporal signal data may moreover be fast, as the emission of a single wave is sufficient, and computationally inexpensive. Accordingly, using plane and/or diverging wave is simpler, as there is no need to compensate any focusing shape. Also, it is faster as it illuminates (i.e. insonifies) lots of points (or regions) in a medium simultaneously.

It may also be possible to use plane and/or diverging waves with different angles. Consequently, a better signal-to-noise ratio may be achieved.

The spatio-temporal signal data may be or may comprise pre-beamformed spatio-temporal signal data and/or radiofrequency (RF) data. Pre-beamformed data may be data that have not been processed by beamforming and/or that will be processed by beamforming. The pre-beamformed data may denote unprocessed data and/or measured data and/or raw data.

The spatio-temporal signal data may comprise a plurality of oscillating and/or periodic signals received from the medium and/or sections of the plurality of oscillating signals. The spatio-temporal signal data may be multi-channel data, wherein . each channel comprises one signal. Each signal of the spatio-temporal signal data and/or the plurality of oscillating and/or periodic signals may comprise at least one oscillating signal component.

Sections of the plurality of oscillating signals may be time sections, for example signal data that fulfill a certain temporal condition and/or one or more instances in time of a respective signal. Time sections may for example be selected based on the assumed wave propagation speed.

The spatio-temporal signal data may be pre-recorded, for example the recording of the spatio-temporal signal data may have happened earlier in time. The recording of the spatio-temporal signal may have happened at a different location, for example in a hospital, while the spatio-temporal signal data may be used in operations performed at another location, for example in a computing device located in data center, in a cloud environment, and/or in a different country or city. The recording of the spatio-temporal signal data may not need to be comprised in the method. The recording of the spatio-temporal signal data may be comprised in the method. Examples of the present disclosure may provide signal post-processing techniques.

The first subset may thus be pre-beamformed data, in particular raw RF data, more in particular sections of the plurality of oscillating ultrasound signals. In contrast, the second may comprise beamformed data, for example image data of the medium (for example one or several pixels).

For example, the first subset may be beamformed according to a predefined beamforming process.

The second subset of beamformed ultrasound data may be obtained in the beamforming process as a function of an assumed wave propagation speed in the medium and assumed reflector properties in the medium, in particular at the spatial region.

The beamformed ultrasound data may be IQ (in-phase and quadrature phase) beamformed ultrasound data.

The result of the evaluation (operation) of the second subset may also be referred to as a figure of merit of the second subset. Said figure of merit may indicate the usability of the second subset for an attenuation estimation of the medium, for example whether it is suitable for a reliable and /or precise attenuation estimation or not. In one example, the figure of merit may correspond to the value of the focusing quality criterion (i.e. a focusing quality value) or may be calculated as a function of the focusing quality criterion.

The figure of merit (which in some cases corresponds to the focusing quality criterion) may also be referred to an attenuation confidence level, as it may be a measure of how valuable the second set is for a precise and/or reliable ultrasound attenuation estimation.

The first subset may be selected in a predefined beamforming process as a function of a first predefined spatial region in the medium.

The second subset may comprise image data of the first predefined spatial region, for example one or several pixels.

In particular, the spatio-temporal signal data of the first subset may be associated with a predefined spatial region in the medium. For example, the spatio-temporal signal data may be associated with a pre-selected spatial region in the medium. The spatial region may correspond to one pixel and/or a plurality of pixels in beamformed image data corresponding to the medium. The pre-selected region may for example constitute a reflector in the medium. The spatio-temporal signal data may be associated with a predefined spatial region in the medium such that the spatio-temporal signal data comprise sections, for example time sections, of the spatio-temporal signal data.

The method may further comprise: determining a first subset of the spatio-temporal signal data as a function of a predefined spatial region in the medium and optionally an assumed wave propagation speed. The spatio-temporal signal data may comprise a plurality of oscillating signals. The first subset may comprise sections of the oscillating signals. The sections may be selected with respect to each other as a function of the spatial region and/or the assumed wave propagation speed, optionally in relation to the locations where the signals are received and/or the signal-readout locations. Selecting the sections may comprise shifting the sections in time and/or delaying the sections. The sections may be shifted and/or delayed relatively to each other depending on the selected spatial region. The sections may be delayed and/or shifted computationally. The sections may be delayed adaptively. The terms shifting and delaying may be used synonymously throughout this disclosure unless denoted differently.

For example, the time sections of different signals of the plurality of signals may be delayed according to time intervals that are selected as a function of the spatial region and/or on the assumed wave propagation speed and/or signal-readout locations and/or the locations where signals are received, for example based on an assumed propagation time differences of a signal arriving from a spatial region at respective different signal readout locations. In such a case, the time intervals of respective signal sections after shifting of one or more from the plurality of oscillating signals may correspond to each other. Alternatively or in addition, time sections of respective ones of the plurality of oscillating signals may be selected such that time intervals of respective ones of the plurality of oscillating signals are time shifted with respect to each other, as a function of the spatial region and/or the assumed wave propagation speed and/ or the signal-readout locations and/or the locations where signals are received, for example based on propagation time differences, corresponding to the assumed wave propagation speed, between respective signal readout locations. Alternatively, or in addition, time sections may be selected based on respective distances between the spatial region and respective signal readout locations and/or based on the respective signal-readout locations and/or the respective locations where signals are received. For example, signals having the same distance, for example the same pulse-echo distance, from the spatial region may not be delayed with respect to each other and/or the time stamps of the respective time intervals may correspond to each other. The operation of determining a first subset of the spatio-temporal signal data may be based on the geometry of a plurality of transducers elements, for example a transducer array, used for receiving the spatio-temporal signal data.

Evaluating the second subset may comprise comparing the figure of merit with a predefined threshold.

The focusing quality criterion may comprise at least one of a coherence property of the first subset, and phase properties of the first subset.

The method may further comprise determining a signal-to-noise ratio (SNR) parameter from the first subset, and evaluating the second subset as a function of the signal-to-noise ratio parameter of the first subset. The operation of determining an SNR parameter may be performed in separate computation to the determination of a focusing quality criterion.

Accordingly, in addition (or as alternative) to determining a focusing quality criterion, the second subset may be evaluated as a function of the signal-to-noise ratio (SNR) parameter of the first subset. Accordingly, the evaluation method may become more reliable, as the SNR can be used as another indicator for the usability of the second subset for ultrasound attenuation estimation.

The focusing quality criterion (in particular the coherence ratio) may comprise a B-mode ratio.

The coherence ratio (and/or B-mode ratio) may be determined by normalizing the beamforming process by an incoherent energy arising from the first predefined spatial region.

The coherence ratio (and/or B-mode ratio) may be determined by determining a coherent sum and a non-coherent sum of the first subset and setting them in a ratio.

The phase properties may comprise at least one of:

- signs,
- phases,
- a sign proportion of the phase properties,
- phase intervals,
- phase evolution states, and
- proportions of phase properties.

Phase properties may comprise any kind of phase properties. Especially, phase properties may comprise at least one of: Signs, phases, a sign proportion, phase intervals, proportions of phase evolution states, phase evolution states, and proportions of phase properties.

The "sign" may indicate whether a signal section (for example one or more instances in time of a signal) is positive or negative. A section of a signal may be one or more instances in time of a signal. In other words, the "sign" may be a binary value being "positive" or "negative". The term "signal section" may be understood as a signal fraction.

The term "sign proportion" may refer to the fraction of signals of a plurality of signals whose signs falls into a certain category, for example by fulfilling a certain criterion, for example being positive or negative, being ascending or descending, and/or combinations thereof. The term "phase intervals" may refer to phases of a plurality of signals being within specified phase intervals, for example between 0° and 180° or between 0° and 90° or between 0° and 45°. The plurality of signals may origin for example from different signal sources (for instance from different transducer elements). In case of only two categories (for example positive sign and negative sign), the term "proportion" may refer to the higher proportion. In case of more than two criteria, the term "proportion" may refer to the highest proportion of a distribution of the categories. The phase properties may be a function of the category distribution. In other examples, the term "proportion" may refer to the lowest proportion of the respective proportions. The following is described assuming that the highest proportion is selected as "the" proportion. However, it is possible to use the lowest proportion instead. In such a case, the word "highest proportion" has to be replaced with "lowest proportion" wherever appropriate, as is readily apparent to the skilled person.

For example, phase evolution states may be defined based on derivatives of the spatio-temporal signal data, for example as a function of time. For example, phase evolution states may denote phase properties such as phases corresponding to "increasing signals" (for example a sinusoidal wave whose phase is between 0° and 90° or 270° and 360°), "decreasing signals" (for example a sinusoidal wave whose phase is between 90° and 270°), "positive sign increasing" (for example a sinusoidal wave whose phase is between 0° and 90°), "negative sign increasing" (for example a sinusoidal wave whose phase is between 270° and 360°), "positive sign decreasing" (for example a sinusoidal wave whose phase is between 90° and 180°), "negative sign decreasing" (for example a sinusoidal wave whose phase is between 180° and 270°), "positive sign" (for example a sinusoidal wave whose phase is between 0° and 180°), "negative sign" (for example a sinusoidal wave whose phase is between 180° and 360°), "minimal change of signal" (for example a sinusoidal wave whose phase is around 90° or around 270°), "maximum change of signal" (for example a sinusoidal wave whose phase is around 180° or 0°/360°), and various other categories readily apparent for the person skilled in the art, such as combinations of above categories or categories denoting selected phase intervals (for example a sinusoidal wave whose signal is between X° and Y°, wherein X and Y may be selected as appropriate). Phase evolution states may be pre-defined.

Using phase properties, for example sign proportions, implies the advantage that the proposed evaluation method may rely on the information of one instance in time of a signal. For example, one instance in time of a signal is sufficient to determine whether the sign of a signal is positive or negative. Examples of the present disclosure thus may provide a more robust evaluation method compared to other methods where knowledge of one instance in time of a signal is not sufficient.

Using evolution states may advantageously allow for a finer categorization of phase properties. This may allow to provide a more precise evaluation method. Using evolution states may require knowledge of respective series of signal data, for example temporal series, for example to determine whether a respective signal is increasing and/or decreasing. Such a method may be more complex and/or less robust. According to examples, broad categories such as sign properties and finer categories such as phase evolution states may be used in combination and/or subsequently. For example, broad categories such as sign proportions may be used in a first iteration and/or in first iterations, and finer categories, for example proportions of phase evolution states and/or phase evolution states may be used in subsequent iterations. Appropriate combination of different phase properties lies in the discretion of the skilled person and may provide methods that are robust, efficient, cheap, fast, and/or precise, optionally at the same time.

The phase properties may be determined by using a predefined function G. The function G may be at least one of:

a function $G(x)$ that is symmetric about the axis $x = 50\%$, configured to maximize at $x=50\%$ and minimize at $x=0\%$ and $x=100\%$ or to minimize at $x=50\%$ and maximize at $x=0\%$ and $x=100\%$,
a bell-shaped function,
a normal distribution function,
a Shannon entropy function.

Evaluating the second subset may further comprise determining a figure of merit of the second subset as a function focusing quality criterion of the first subset. In other words, the evaluation result, i.e. the evaluated second subset may also be referred to as a figure of merit. In one example, the figure of merit may correspond to the focusing quality criterion, i.e. the determined value of the focusing quality criterion.

The function G may provide the highest figures of merit if either all phase properties fall into a certain category or if none fall into a certain category, for example either all signs being positive or all signs being not positive (for example negative). The figure of merit may denote a purity of the phase properties, for example a purity of signs, which is for example maximum if all signs fall into one category. For example, the figure of merit may be a purity measure of phase categories. The figure of merit may be based on the distribution of categories. For example, the figure of merit may be a negative Shannon entropy of the category distribution. The figure of merit may be maximized as the category distribution concentrates itself on a single category.

A figure of merit may be for example a quantitative and/or qualitative measure of the evaluation result. A figure of merit may also be referred to as an evaluation value. The figure of merit may allow comparing different evaluated second sets, in order to evaluate them. The figure of merit may for example be an entropy, for example more particularly a negative Shannon entropy, of phase properties, for example an entropy of sign proportions, for example an entropy of the ratio and/or the proportion of positive signs and/or negative signs.

Evaluating the second subset may comprise comparing the focusing quality criterion of the respective first subset with a predefined range. The predefined range may be defined by a predefined upper limit and/or a predefined lower limit.

The second subset may be evaluated to qualify for (or to be useable for) the purpose of ultrasound attenuation estimation in a medium, in case the focusing quality criterion (in particular the coherence ratio) of the respective first subset is within the predefined range.

The second subset may be evaluated to be not useable for the purpose of ultrasound attenuation estimation in a medium, in case the focusing quality criterion of the respective first subset is outside the predefined range.

Beamforming a first subset may comprise beamforming a plurality of first subsets of ultrasound spatio-temporal signal data to obtain a respective plurality of second subsets of beamformed ultrasound data, wherein the plurality of first subsets is associated with different spatial regions in the medium.

Determining a focusing quality criterion may comprise determining for each of a plurality of first subsets a focusing quality criterion.

Evaluating the second subset may comprise evaluating a plurality of second subsets as a function of the focusing quality criteria of the respective first subsets, and optionally generating a focusing quality map based on the evaluated plurality of second subsets. Accordingly, the map may consist of a plurality of spatial regions or the medium, each of which being represented by a respective second set (for example a pixel or a group of pixels). Each region of the map may be assigned with at least one figure of merit, for example a focusing quality value.

The focusing quality may comprise in addition (for example as superimposed map) or as alternative to the focusing quality map a segmentation map. The segmentation map may segment (or select) at least one evaluated second subset as a function of the respective focusing quality criterion. The segmented or selected second subset may be determined to be suitable for the purpose of attenuation estimation.

Accordingly, the present disclosure may also relate to a method of segmenting a second subsets based on their focusing quality values.

The method may further comprise evaluating an area in the focusing quality map comprising a plurality of adjacent second subsets as a function of a difference between the focusing quality criteria of the respective first subsets. The focusing quality map may also be referred to as an attenuation confidence map, or validation map.

Determining a focusing quality criterion may comprise determining a focusing quality criterion from the first subset and additionally from at least one further first subset associated with a neighboring spatial region with respect to the first predefined spatial region.

The method may further comprise determining a speckle statistic of the second set. The second set may optionally comprise IQ beamformed ultrasound data.

The present disclosure further relates to a method of estimating an ultrasound attenuation property in a medium. The method comprises: a method of evaluating ultrasound data according to the present disclosure, selecting at least one evaluated second subset as a function of the respective focusing quality criterion, and estimating the ultrasound attenuation property by applying a predefined attenuation estimation method to the selected second subset.

For example, the ultrasound attenuation property may be estimated based on an amplitude variation across the depth direction of the spatial region represented by the second subset. In this regard it is referred to for example EP3936891A1 filed by the same applicant, which disclosure is incorporated herein by reference. However, also other attenuation estimation methods are possible.

In general, it is noted that the method of the present disclosure of evaluating ultrasound data may also have further purposes beside the purpose of ultrasound attenuation estimation. For example, the evaluation (i.e. the determined focusing quality criterion) may also serve to estimate at least one ultrasound backscattering coefficient of the medium. Accordingly, the evaluated second subset may be used to estimate the backscattering coefficient. For example, an ultrasound attenuation estimation may be carried out, based on which the at least one backscattering coefficient is estimated. In another example, the at least one backscattering coefficient may be estimated directly based on the evaluated second subset (without an explicit determination of any estimated ultrasound attenuation property).

Accordingly, the present disclosure may further relate to a method of estimating an ultrasound backscattering coefficient in a medium, comprising: a method of evaluating ultrasound data according to the present disclosure or a method of estimating an ultrasound attenuation property according to the present disclosure, selecting at least one evaluated second subset as a function of the respective focusing quality criterion or (in case the present method is based on a method of estimating an ultrasound attenuation property) as a function of an estimated ultrasound attenuation property, estimating the backscattering coefficient by applying a predefined backscattering estimation method to the selected second subset.

An exemplary backscattering estimation method is described by Lin Xin Yao, James A. Zagzebski, Ernest L. Madsen: Backscatter coefficient measurements using a reference phantom to extract depth-dependent instrumentation factors, Ultrasonic Imaging, Volume 12, Issue 1, 1990, Pages 58-70, ISSN 0161-7346. According to this work, a relative processing method for determining the backscatter coefficient and the attenuation coefficient is proposed. This method involves comparison of echo data from a sample with data recorded from a reference phantom whose backscatter and attenuation coefficients are known. A time domain processing technique is used to extract depth and frequency dependent signal ratios for the sample and the reference phantom. The attenuation coefficient and backscatter coefficient of the sample are found from these ratios. The method is tested using tissue-mimicking phantoms with known scattering and attenuation properties. Accordingly, the backscattering estimation method may be optimized by the method of the present disclosure, for example by selecting a suitable region based on the focusing criterion and/or by permitting a reliable and precise attenuation estimation.

For example, the backscattering estimation method may comprise estimating an ultrasound energy response of the medium, and estimating the backscattering coefficient as a function of the ultrasound energy response. The ultrasound energy response may be received from the medium in response to an emitted ultrasound pulse (for example a plane and/or diverging wave emitted by a probe into the medium). The pulse may be that one used for establishing the ultrasound spatio-temporal signal data used for evaluating the second subset.

In general, different mediums may have different backscattered energy responses. Accordingly, the mediums may also have different ultrasound backscattering coefficients, respectively. For example, different attenuation properties may influence the energy of the response and hence the backscattering coefficient. In other words, the backscattered energy may be impacted by both the attenuation and backscattering coefficient.

Thus, a reliable and precise attenuation estimation for a medium or a region of interest in a medium may advantageously be taken into account, when a backscattering coefficient is estimated.

In other words, estimating the backscattering coefficient of the medium advantageously requires to reliably and precisely know the attenuation properties of the medium, what in return may for example require to generate a focusing quality map to identify suitable regions for the attenuation estimation.

The present disclosure further relates to a computer program comprising computer-readable instructions which when executed by a data processing system cause the data processing system to carry out the method according to any one of preceding method claims.

The present disclosure further relates to a system for evaluating ultrasound data for the purpose of ultrasound attenuation estimation in a medium, the medium being associated with ultrasound spatio-temporal signal data received from the medium in response to at least one ultrasound plane and/or diverging wave emitted into the medium. The system comprises a processing unit configured to:

- beamform a first subset of ultrasound spatio-temporal signal data to obtain a second subset of beamformed ultrasound data,
- determine a focusing quality criterion from the first subset, and
- evaluate the second subset as a function of the focusing quality criterion of the first subset.

The system may furthermore be configured to perform any of the method features or operations described above.

It is intended that combinations of the above-described elements and those within the specification may be made, except where otherwise contradictory.

It is to be understood that both the foregoing general description and the following detailed description are exemplary and explanatory only, are provided for illustration purposes and are not restrictive of the disclosure, as claimed.

The accompanying drawings, which are incorporated in and constitute a part of this specification, illustrate examples of the disclosure and together with the description, and serve to support and illustrate the principles thereof.

BRIEF DESCRIPTION OF THE DRAWINGS

Fig. 1 shows a schematic drawing of an ultrasound system according to examples of the present disclosure;

Fig. 2 shows a flowchart providing an overview of the flowcharts of fig. 3a and 3b;

Fig. 3a shows a flowchart f1 of an exemplary method of evaluating ultrasound data for the purpose of ultrasound attenuation estimation in -a medium according to the present disclosure, the method may be implemented in the system of Fig. 1;

Fig. 3b shows a flowchart f2 of an exemplary method of estimating an ultrasound attenuation property in a medium according to the present disclosure, which may for example be implemented in the system of Fig. 1 and/or be based on the method of Fig. 3a;

Fig. 4a shows an exemplary ultrasound image with a selected region of interest, for which ultrasound data shall be evaluated for the purpose of ultrasound attenuation estimation;

Fig. 4b shows a focusing quality map for the selected region of interest of fig. 4a according to an example of the present disclosure.

Fig. 5 shows an ultrasound image with a focusing quality map in a grid form according to an example of the present disclosure; and

Fig. 6 shows an example of evaluating ultrasound data based on phase properties for the purpose of ultrasound attenuation estimation.

DESCRIPTION OF THE DRAWINGS

Reference will now be made in detail to examples of the disclosure, examples of which are illustrated in the accompanying drawings. Wherever possible, the same reference numbers will be used throughout the drawings to refer to the same or like parts.

In general, a medical diagnostic apparatus is known from for example US 10,932,750 B2, which scans the inside of the living body to create a tomographic image of an organ or the like using attenuation quantification. However, according to the document, many different tissues exist in an actual living body, whereby the living body has a complicated structure. Thus, there are few and usually small portions constituted of uniform scatterers. For example, in the liver, even if a portion looking like a uniform speckle in the range of several centimeters seems to be present, when a focusing range is widened, different structures can be visually recognized, such as blood vessels, abdominal wall, and/or a gall bladder, enter a focusing range.

The document US 10,932,750 B2 thus proposes that for quantification of the attenuation amount with high accuracy, in a clinical field, there is required a procedure in which a cross section being as inconspicuous in a structure as possible is set, and a uniform region is selected from the set cross section to set the analysis target region. In this regard, a tissue characterization (for example, the attenuation constant) at a plurality of positions except for an estimated structure position is calculated based on B-mode data for attenuation quantification.

However, the proposed method has several drawbacks. First, the method may only exclude or include positions into/from the data to be used for attenuation estimation, i.e. act as a mere binary filter. As a consequence, any position where a structure is estimated, is not taken into account. Second, all positions where no structure is estimated, are equally taken into account, without considering any other differences between these positions. Accordingly, other characteristics of the medium, which may be relevant to qualify the scanned data for a reliable and/or precise attenuation estimation, are not taken into account.

The system and method described herein are related to technologies for evaluating ultrasound data for the purpose of ultrasound attenuation estimation in a medium, in particular for medical imaging. In particular, the method is suitable for processing signal data of a medium scanned by a transducer device. For example, the method may be used in a device such as for instance an ultrasound system.

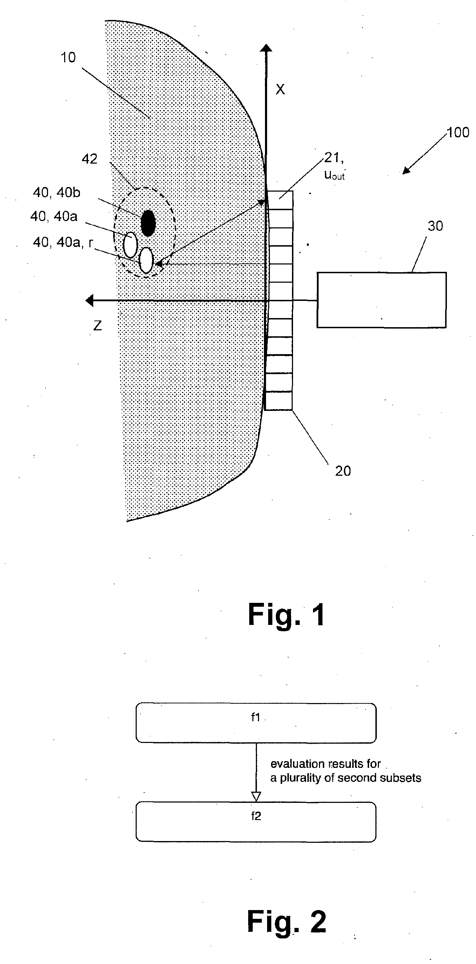

Fig. 1 shows a schematic drawing of an ultrasound system according to examples of the present disclosure. The system 100 shown on Fig. 1 may be adapted for attenuation estimation in a medium 10, and in particular for evaluating ultrasound data for the purpose of ultrasound attenuation estimation. The system may further be adapted for ultrasound imaging instance. The medium may comprise living tissues and in particular human tissues of a person, for example a sporty professional and/or a patient. The system may include for instance:

- A plurality of transducer elements 21. The transducer elements may be configured to transmit (a) a pulse into the medium (for example in the form of a planar wave) and/or to receive (b) a plurality of signals from the medium, optionally in response to transmitting (a) the pulse into the medium. A transducer array comprising a plurality of transducer elements 21 may be used. For example, a linear array 20 may be provided typically including a few tens of transducer elements (for instance 100 to 300) juxtaposed along an axis X (horizontal or array direction X) as already known in usual probes. In this example, the array 20 is adapted to perform a bidimensional (2D) imaging of the medium 10, but the array 20 could also be a bidimensional array adapted to perform a 3D imaging of the medium 10. The transducer array 20 may also be a convex array including a plurality of transducer elements aligned along a curved line. The same transducer element(s) may be used to transmit a pulse and receive the response, or different transducer elements are used for transmission and reception. There may be one or more emitting transducer elements and a plurality of receiving transducer elements. In a further alternative, only a single transducer element may be used which receives a plurality of signals which have different spatial properties (for example originating from different spatial regions). The transducer element may for instance be electronically or physically moveable;
- an electronic bay 30 controlling the transducer array and acquiring signals therefrom.

The system may further include a microcomputer (not shown) for controlling the electronic bay 30 and/or for example for sending data to a server, to an artificial intelligence (AI) entity, to a dedicated workstation, presenting data, displaying/viewing images obtained from the electronic bay (in a variant, a single electronic device could fulfil all the functionalities of the electronic bay 30 and the microcomputer).

According to further examples, the system 100 may typically include at least one processing unit (or processor) and memory. In examples, the processor and memory unit may be incorporated into the system or may be a computer or computer communicatively linked thereto. Depending on the exact configuration and type of computing device, memory (storing, instructions to evaluate ultrasound data or otherwise perform the methods described herein) may be volatile (such as RAM), nonvolatile (such as RAM, flash memory, etc.), or some combination of the two. Further, the system 100 may also include storage devices (removable and/or non-removable) including, but not limited to, magnetic or optical disks or tape. Similarly, the system 100 may also have input device(s) such as keyboard, mouse, pen, voice input, etc. and/or output device(s) such as a display, speakers, printer, etc. Also included in the environment may be one or more communication connections, such as LAN, WAN, point to point, etc. In embodiments, the connections may be operable to facility point-to-point communications, connection-oriented communications, connectionless communications, etc.

The system 100 typically includes at least some form of computer readable media. Computer readable media can be any available media that can be accessed by processing unit (or processor) or other devices comprising the operating environment. By way of example, and not limitation, computer readable media may comprise computer storage media and communication media. Computer storage media includes volatile and nonvolatile, removable and non-removable media implemented in any method or technology for storage of information such as computer readable instructions, data structures, program modules or other data. Computer storage media does not include communication media.

Communication media embodies computer readable instructions, data structures, program modules, or other data in a modulated data signal such as a carrier wave or other transport mechanism and includes any information delivery media. The term "modulated data signal" means a signal that has one or more of its characteristics set or changed in such a manner as to encode information in the signal. By way of example, and not limitation, communication media includes wired media such as a wired network or direct-wired connection, and wireless media such as acoustic, RF, infrared, microwave, and other wireless media. Combinations of the any of the above should also be included within the scope of computer readable media.

The system 100 may be a single computer operating in a networked environment using logical connections to one or more remote computers. The remote computer may be a personal computer, a server, a router, a network PC, a peer device or other common network node, and typically includes many or all of the elements described above as well as others not so mentioned. The logical connections may include any method supported by available communications media. Such networking environments are commonplace in offices, enterprise-wide computer networks, intranets and the Internet.

The transducer elements 21 may comprise piezo-crystals and/or other components that may be configured to generate and/or record and/or receive signals. The terms transducer and transducer elements may be used synonymously throughout this disclosure unless denoted differently.

Transducer elements 21 may be configured to generate and/or record and/or receive signals, optionally ultrasonic signals. Transducer elements 21 and/or electronic bay 30 and/or the microcomputer may be configured to determine phase properties of spatio-temporal signal data.

The axis Z on figure 1 is an axis perpendicular to the axis X, for example in the depth direction of the examined medium. This direction is designated in the present document as a vertical or axial direction.

The medium 10 may comprise a spatial region 40. The spatial region 40 may be predefined and/or may be selected during operation of methods according to examples of the present disclosure. The medium 10 may comprise a plurality of spatial regions 40.

The medium 10 may optionally comprise one or more regions of interest 42. Spatial regions 40 optionally may be comprised only in one or more regions of interest 42. Spatial regions 40 may be located in the medium at various positions not necessarily restricted to specific regions of interest 42. The whole medium or a part of the medium may be the region of interest 42. For example, only regions corresponding to regions in beamformed image data corresponding to the medium and/or the region corresponding to the entire beamformed image data may be regions of interest 42. For example, it may be most desired to estimate ultrasound attenuation in the region of interest 42. For this purpose, it may be advantageous to evaluate ultrasound data of different spatial regions 40 in the region of interest 42, in order to select those which are suitable for a precise and/or reliable attenuation estimation.

For example, the region of interest 42 may comprise one or several spatial regions 40b which have inhomogeneities of the medium, for example different levels of stiffness of its tissue(s). In this case, the spatial regions 40b are not or less suitable for a reliable and/or precise ultrasound attenuation estimation. At the same time, the region of interest 42 may comprise one or several spatial regions 40a which have no or less inhomogeneities of the medium. In this case, the spatial regions 40a may be selected for an ultrasound attenuation estimation. The evaluation and selection methods are described in more detail in the examples of fig. 2 to 5. The method optionally may produce ultrasound images of the medium 10 and/or of the region of interest 42 of the medium 10 and/or may send data to a dedicated server or working station.

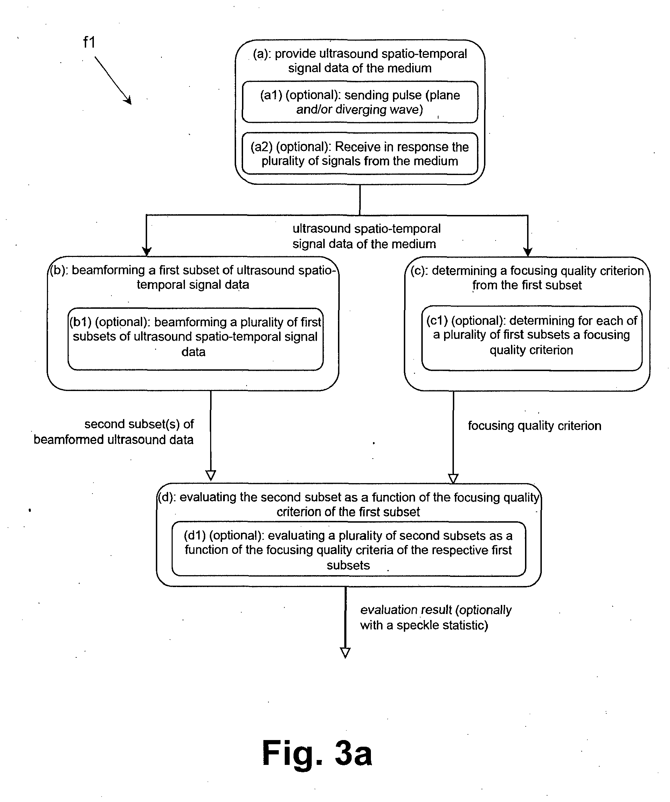

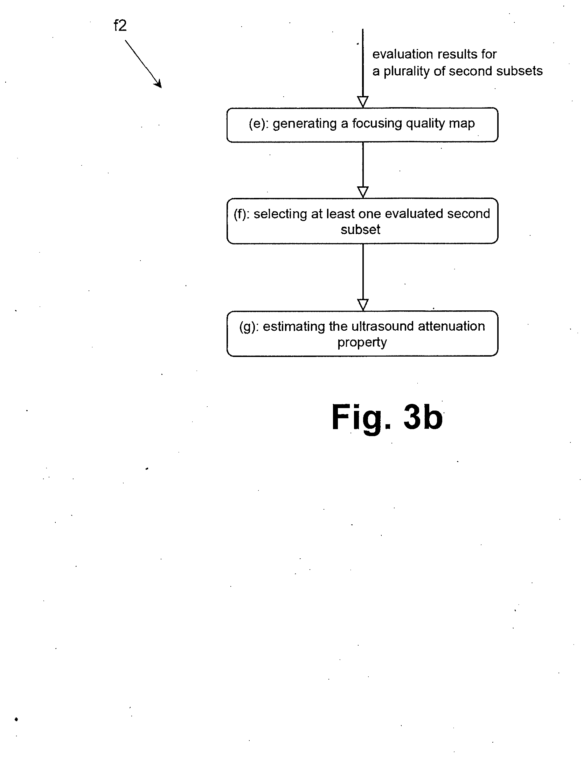

Fig. 2 shows a flowchart providing an overview of the flowcharts of fig. 3a and 3b. The method may comprise operations according to a first flowchart f1 (as shown in more detail in fig. 3a) and operations according to a second flowchart f2 (as shown in more detail in fig. 3a). In particular, the output of the operations according to flowchart f1 may be the input for the operations according to flowchart f2. Said output may be an evaluation result for a plurality of subsets, as explained in more detail in context of fig. 3a and 3b.

Fig. 3a shows a flowchart f1 of an exemplary method of evaluating ultrasound data for the purpose of ultrasound attenuation estimation in a medium according to the present disclosure, the method may be implemented in the system of Fig. 1.

In an operation (a) ultrasound spatio-temporal signal data of the medium is provided. The spatio-temporal signal data may be received from the medium in response to at least one ultrasound plane and/or diverging wave emitted into the medium.

For example, the method may comprise an optional operation of transmitting (a1) a pulse into the medium. For instance, the transmission operation may comprise insonification of the medium with an ultrasonic plane and/or diverging wave. More in particular, during transmission operations a plurality of ultrasonic plane and/or diverging waves may be transmitted into the medium 10 and on particular into a spatial region 40.

Generally, a wave may correspond to the wavefront generated by one or several transducer elements (i.e. by respectively emitted pulses). The wave may be controlled by means of emission delay between the different used transducer elements. Examples comprise a plane wave, a focused wave and a divergent wave. A beam may correspond to the physical area insonified by the wave (for example in the medium). Hence, the beam may be related to the wave but may have less or no temporal notion. For example, it may be referred to a beam when the depth of field of a focused beam is of interest.

In an optional operation (a2), a plurality of signals may be received, optionally in response, from the medium by the plurality 20 of transducer elements 21. The plurality of signals may comprise backscattered echoes of the transmission of operation (a1). The response sequence may also be referred to as spatio-temporal data and/or signal data, in particular ultrasound signal data and/or RF and/or IQ signal data. The signal data may be in the time domain, more in particular in a spatio-temporal domain, as for example described in more detail below. In one example, the response sequence may be processed by bandpass filtering, in order to keep only one or several frequency ranges.

It is noted that operations (a1) and (a2) are optional, as they may also be carried out by any other system than the system used for operations (b) to (d) and optional operations (e) to (g) and/or at another time. It is also possible that the spatio-temporal signal data are pre-stored, and for example provided by/read on a data storage, a communication interface, etc.

In an operation (b) a first subset of ultrasound spatio-temporal signal data is beamformed to obtain a second subset of beamformed ultrasound data. Optionally, a plurality of first subsets of ultrasound spatio-temporal signal data to obtain a respective plurality of second subsets of beamformed ultrasound data. For example, the plurality of first subsets may be associated with different spatial regions (cf. for example spatial regions 40 in fig. 1) in the medium. In another example, the beamformed ultrasound data may be image data, and/or the second subset may be one or several image pixels or voxels.

In an operation (c), a focusing quality criterion from the first subset is determined. Optionally, for each of a plurality of first subsets a focusing quality criterion is determined. The plurality of first subsets may correspond to that one of the optional operation (b1). Accordingly, for each spatial region (associated with or represented by a respective first subset), a focusing quality criterion may be determined.

An exemplary method of determining a focusing quality criterion is described below in more detail.

It is noted that operations (b) and (c) may be carried out simultaneously. This may accelerate the method of the present disclosure and for example be advantageous when carrying out the method in real-time. In this case, a further operation (not shown in fig. 1) of selecting a first set (as described above) as a function of a predefined spatial region may be carried out after operation (a) and before operations (b) and (c).

For example, data in the first subset corresponding to a specific spatial region (for example spatial region 40) in the medium may be determined based on the assumed propagation time of a signal between the spatial region 40 and respective transducer elements 10. The assumed propagation times may be determined based on the location of the spatial region 40 and the location of the plurality of transducers and/or each respective transducers, optionally in relation to the location of the spatial region 40, and optionally an assumed wave propagation speed. Determining a first subset of the spatio-temporal signal data as a function of a predefined spatial region 40 in the medium 10 and as a function of the assumed wave propagation speed may be based on the geometry of the plurality 20 of transducers 21. It is noted that the spatial region may be associated with a pixel or a plurality of pixels in beamformed image data (i.e. the second subset).

However, operations (b) and (c) may also be carried out or successively. For example, operation (b) may be carried out before operation (c). In this case, the operation of selecting a first set (as described above) as a function of a predefined spatial region may be part of operation (b), i.e. of the beamforming process.

In an operation (d), the second subset is evaluated as a function of the focusing quality criterion of the first subset. Optionally, a plurality of second subsets may be evaluated as a function of the focusing quality criteria of the respective first subsets.

The output of operation (d) may be referred to as an "evaluation result", an "evaluated second subset", "focusing quality value" or a "figure of merit", as described above.

Optionally, operation (d) may also include determining a speckle statistic. In this case it is desirable that the second set optionally comprises IQ beamformed ultrasound data and optionally that a plurality of second subsets is evaluated. The speckle statistic may provide further valuable information to evaluate the usability of the second set for a reliable and/or precise attenuation estimation. However, the speckle statistics may also be provided by (i.e. it may be) the focusing quality criteria of the second subsets.

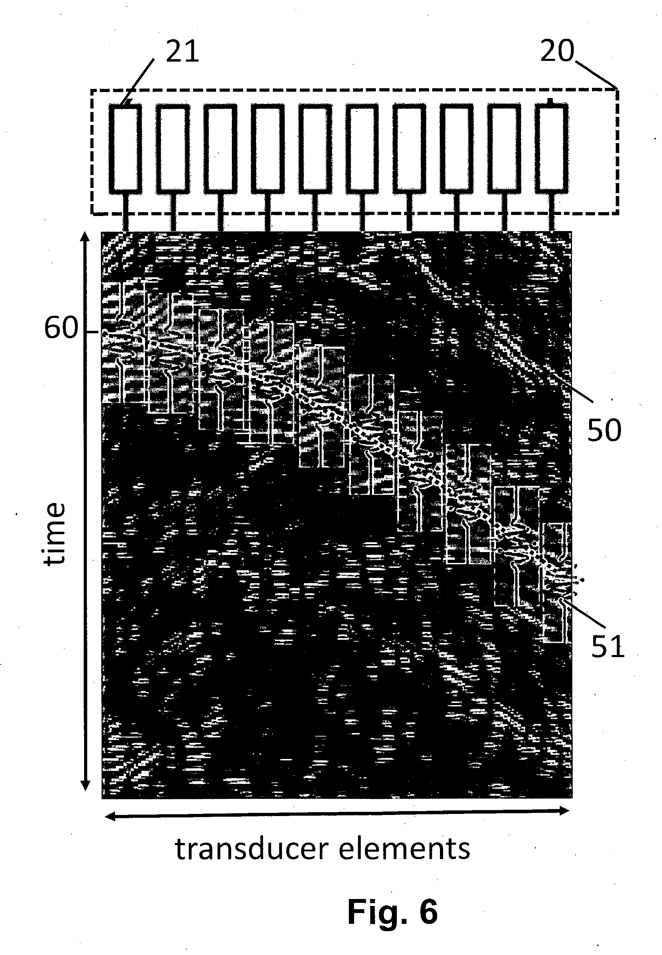

Different exemplary methods of determining a focusing quality criterion are described below. As described, the focusing quality criterion may be determined based on a coherence property, in particular a B-mode ratio. However, it is also possible that the focusing quality criterion is determined based on for example phase properties of the first subset, as described in context of fig. 6.

In order to determine the focusing quality criterion based on a coherence property, a medium may be insonified with $N_{\theta_{in}}$ plane waves of angle $\theta_{in}$ (also cf. operation (a1)). For each transmitting plane wave, the backscattered echoes at time t are recorded by $N_{u_{out}}$ transducers located at $\boldsymbol{u}_{out} = \{u_{out}^x, u_{out}^z\}$ (also cf. operation (a2). The receive IQ data may be noted as $R(\theta_{in}, u_{out}, t)$, i.e. as a complex matrix.

Conventional plane wave coherent compounding aims to coherently sum echoes that are assumed to come from the same location. To do so it is assumed that:

> The medium is homogeneous with a constant speed of sound (a more complex speed of sound model may be chosen but require a predefined time of flight computation).
> - All the backscattered echoes of interest results from a single scattering process.
> - The medium is composed of unresolved scatterers that scatter incoming wave in every direction.

For a given location $r = \{x, z\}$ (cf. fig. 1) the required time of flight $\tau(\theta_{in}, \boldsymbol{u}_{out})$ may be determined for each incident wave of angle $\theta_{in}$ to reach the location r and to come back to the element located at $\boldsymbol{u}_{out}$ (cf. fig. 1). Consequently, the result of the beamforming process may be express as (cf. eq. 1):

$$b(\boldsymbol{r}) = \sum_{\theta_{in}(r)} \sum_{u_{out}(r)} R(\theta_{in}, u_{out}, \tau(\theta_{in}, \boldsymbol{u}_{out})) \times \alpha(\theta_{in}, u_{out}, \boldsymbol{r}) \qquad (1)$$

Where $\theta_{in}(r)$ is the ensemble of plane wave used during the beamformer process at point $\boldsymbol{r}$; $u_{out}(r)$ may be the ensemble of received element used during the beamformer process and $\alpha(\theta_{in}, u_{out}, r)$ represent an apodization coefficient, wherein (cf. eq. 2):

$$\sum_{\theta_{in}(r)} \sum_{u_{out}(r)} \alpha(\theta_{in}, u_{out}, \boldsymbol{r}) = 1< \qquad (2)$$

The focusing quality criterion may be determined based on a coherence property, in particular a B-mode ratio, as also referred to by:
Mallart, Raoul and Mathias Fink. "Adaptive focusing in scattering media through sound-speed inhomogeneities: The van Cittert Zernike approach and focusing criterion." Journal of the Acoustical Society of America 96 (1994): 3721-3732.
In the present disclosure the concept of Mallart et.al. may be extended to the case of plane wave imaging.

B-mode ratio consists in normalizing the conventional beamforming process by the incoherent energy arising from the location of interest (cf. eq. 3):

$$ratio(\boldsymbol{r}) = \frac{\left| \sum_{\theta_{in}(r)} \sum_{u_{out}(r)} R(\theta_{in}, u_{out}, \tau(\theta_{in}, u_{out})) \times \alpha(\theta_{in}, u_{out}, r) \right|^2}{\sum_{\theta_{in}(r)} \sum_{u_{out}(r)} |R(\theta_{in}, u_{out}, \tau(\theta_{in}, u_{out})) \times \alpha(\theta_{in}, u_{out}, r)|^2} \qquad (3)$$

wherein r corresponds to the pre-beamformed data for one pixel (i.e. a first subset).

The calculated B-mode ratio indicates whether the phases of the selected signals are coherent (i.e. good in phase / aligned) or not. Examples are described below in context of fig. 6 and 7.

According to Mallart et.al., in pure speckle this ratio tends toward 0.67. For a point like bright reflector in the medium, it tends toward 1 and when aberrations arise and/or reverberation and/or noise, this ratio decreases.

Consequently, by filtering out (i.e. not selecting in operation (f) of fig. 3b) relatively low values (for example < 0.45) and relatively high values (for example > 0.9) it may be possible to select a well beamformed speckle region.

In this context it is referred to an exemplary scenario, wherein in a medium a bone is in front of soft organ (i.e.

in. a z- or depth-direction in fig. 1): The bone is a strong reflector (i.e. a specular reflector) and hence reflects an increased amount of energy. Hence, the bone may be very bright on. a b-mode image. In contrast, due to the increased reflection of the bone, the organ will be "shaded" by the bone and hence will be relatively dark in the image. An attenuation estimation in this scenario would calculate a very strong attenuation, as it would take the high energy of the bone in comparison to the dark area of the organ. Even an attenuation estimation which only takes into account the'region behind the bone (but not the bone itself) would not be suitable, as the region directly behind the bone is shaded and is hence too dark. An attenuation estimation in this scenario would calculate a very low or even no attenuation, as the medium farer away of the bone would not be shaded anmore and hence be brighter. Such problematic regions can be identified by the B-mode ratio.

In view of these problems, it may be advantageous to filter out (i.e. disregarded/ignored) regions (i.e. second subsets) with relatively high B-mode ratio values, for example bones or other regions with an increased stiffness (which may be specular reflectors).

Moreover, it may be advantageous to filter out (i.e. disregarded/ignored) regions (i.e. second subsets) with relatively low B-mode ratio values. Relatively low values may represent noise (for example no speckle, i.e. no scatterers present in the region). Such a region may be badly focused in the beamforming process and thus only provide noisy information. The region is hence not suitable for estimating the attenuation reliably and/or precisely, meaning here not a problem of inhomogeneity but of noise. More generally, a low ratio value may mean at least one of: a noisy region, an increased aberration in this region, and a shaded region, all of which are not suitable for attenuation estimation.

Additionally, an area which comprises a combination of relatively high and relatively low B-mode ratio values may be filtered out. Such an area may be understood as comprising inhomogeneous regions.

Accordingly, a suitable area, which may be selected for attenuation estimation, may have regions with a B-mode ratio (or another type of focusing quality criterion) within a predefined range. The predefined range may be defined by a first minimum threshold (for example < 0.2) and a second maximum threshold (for example > 0.8). The B-mode ratio values of the regions may be all close to a desirable ratio level, for example of 0.67 advantageously. In other words, they may be within a smaller second range which defines a predefined tolerance range (for example +/-0.3) around a desirable ratio level.

Fig. 3b shows a flowchart f2 of an exemplary method of estimating an ultrasound attenuation property in a medium according to the present disclosure. This method may for example be implemented in the system of Fig. 1 and/or be based on the method of Fig. 3a. In particular, the output of operation (d) of the method of fig. 3a may be the input for operation (e) of fig. 3b. It is noted that the method of fig. 3b comprises merely optional operations of the method of the present disclosure.

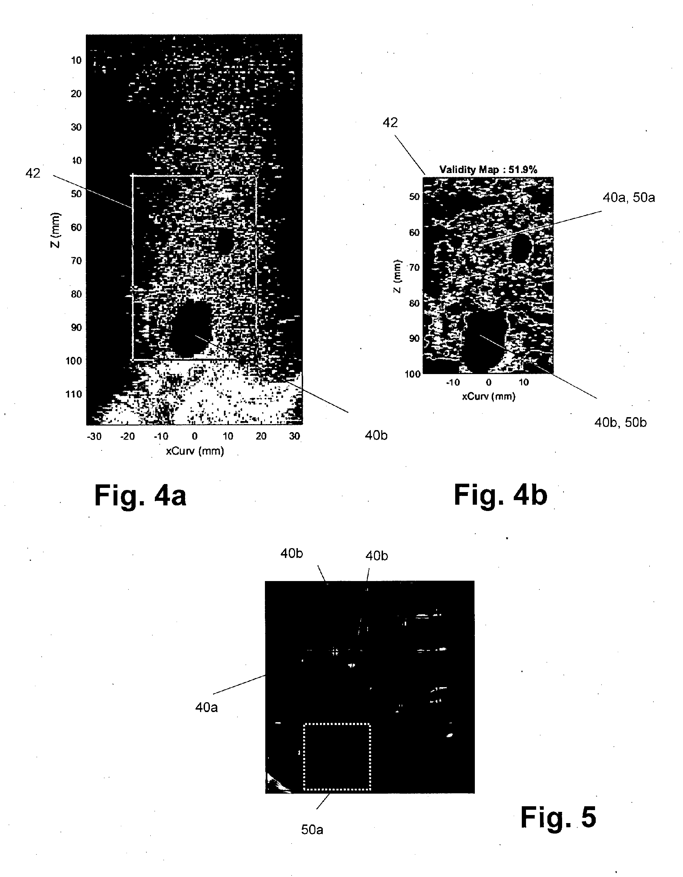

In an optional operation (e) a focusing quality map may be generated based on the evaluated plurality of second subsets. Examples of maps are shown in fig. 4a, 4b and 5. The map may for example have a resolution corresponding to an ultrasound image (for example a B-mode (brightness mode) image) of the medium or the region of interest (cf. fig. 4a, 4b), or a lower resolution (cf. fig. 5). The map may have a size and/or comprise information of the complete medium or at least of the region of interest. The map may be shown to the user on a display device connected to the system, for example adjacent to an ultrasound image or superimposing an ultrasound image. In this way, a user of the system may be informed which areas of the imaged medium may be suitable (i.e. have a high confidence level) for an attenuation estimation. The system may also provide a user interface, where the user can manually select an area (i.e. one or several second subsets) for attenuation estimation.

Alternatively or additionally, the optional operation (f) of selecting at least one of the evaluated second subsets may be carried out automatically by the system, for example by selecting one or several second subsets having figures of merit above a predefined confidence threshold or by comparing the figures of merit with each other and selecting a predefined number of most suitable ones of ultrasound attenuation estimation,

In an optional operation (g) an ultrasound attenuation property is estimated by applying a predefined attenuation estimation method to the selected second subset. An ultrasound attenuation estimation method is known from for example from patent application EP3936891A1 filed by the same applicant, which disclosure is incorporated herein by reference.

Fig. 4a shows an exemplary ultrasound image with a selected region of interest 42, for which ultrasound data shall be evaluated for the purpose of ultrasound attenuation estimation. The evaluation may be done using the method of the present disclosure. The ultrasound image may show a medium and in particularl a region of interest 42 in the medium. The ultrasound image may be for example a B-mode image, in particular constructed based on one or several planar waves insonifying the medium. It is noted that the region of interest 42 is merely an example. It is also possible that an evaluation is desired for another region of the ultrasound image or for the several regions or for the complete image.

Fig. 4b shows a focusing quality map (i.e. a validity map) for the selected region of interest 42 of fig. 4a according to an example of the present disclosure. The focusing-quality may provide for each image pixel a focusing quality value (determined by for example operation c1). However, it may also comprise in addition (for example as superimposed map) or as alternative a segmentation map (determined for example in the selection operation f), as an example is explained in more detail below. The example of Fig. 4b shows both a map of focusing quality value per single pixels and an overlying segmentation map (cf. grey areas) which segments suitable regions 40a from not suitable regions 40b.

In the region of interest 42, the medium may comprise a spatial region 40b (represented by one or several pixels) which is not suitable for ultrasound attenuation. The region 40b may be identified based on for example a relatively low B-mode ratio. Accordingly, an area 50b comprising said region 40b may be disqualified from being used to estimate an ultrasound attenuation. The method of the present disclosure may thus comprise an automated process (for example in operation (f)) of selecting an area 50a based on the focusing quality map. The selected area 50a may for example comprises only suitable regions 40a (for example only pixels with acceptable B-mode ratios).

In one example the focusing quality map may be in the form of or additionally comprise a segmentation map corresponding to the resolution of the ultrasound image. This map may indicate, which regions are suitable (or more suitable) for ultrasound attenuation estimation, and which are not suitable (or less suitable). The map may hence be for instance a binary classifier distinguishing between suitable and not suitable regions. In the example of fig. 4b, the suitable regions are shown in grey color. In addition, a percentage of suitable regions in the selected region of interest 42 may be calculated based on the map (in the example of fig. 4b 51,9%). Such information (i.e. the focusing quality map and/or the percentage value) may be useful for a user to decide, whether an ultrasound attenuation estimated for the selected region 42 is reliable.

The focusing quality map may be shown to the user on a display device connected to the system, for example adjacent to an ultrasound image or superimposing an ultrasound image. In this way, a user of the system may be informed which areas of the imaged medium may be suitable (i.e. have a high confidence level) for an attenuation estimation. The system may also provide a user interface, where the user can manually select an area 50a for attenuation estimation.

It is noted that the selected area 50a may have any shape. For example, the area may be determined by a segmentation process (for example in operation (f)), in which all suitable regions are selected (for example a plurality of adjacent). The area may also have an elongated shape extending in a depth direction. Such an extension in the depth direction may be advantageous for a precise attenuation estimation, as described for example in patent application EP3936891A1 filed by the same applicant. For example, the ultrasound attenuation property may be estimated based on an amplitude variation across the depth direction of the spatial region represented by the second subset.

For example, the liver is a large organ full of blood. On standard B-mode images a lot of veins can be observed. Thus, it can be difficult to find a large homogeneous region. In addition, when using a focusing quality map, areas can be observed that are not well reconstructed due to aberrations, reverberation and/or multiple scattering (which cannot be deferred from the B-mode image data). Hence, the quality map may help a user to manually select a suitable area 50a, or an algorithm to automatically select the area 50a.

Fig. 5 shows an ultrasound image with a focusing quality map in a grid form according to an example of the present disclosure. In this example, the regions selected (or not selected) may not be represented by single image pixels but by fields of a predefined pattern, for example in a grid form.

In other words, the pattern may have a grid form. Each region 40(a/b) may have the same size, and/or each region may represent a different position in the medium according to the pattern.

Accordingly, a predefined pattern may be superimposed onto the ultrasound image. The single fields defined by the pattern may identify suitable regions 40a and not suitable regions 40b. Based on an area of suitable regions 40a it is possible to determine an area 50a for ultrasound attenuation estimation. The selected area 50 may comprise a plurality of adjacent regions 40a.

This exemplary method may have the advantage that less calculations have to be made for determining a suitable area 50a and hence the method may be faster and/or require less computing power. For example, for every field of the pattern (which corresponds to a plurality of pixels of the ultrasound image), it may be adapted to determine one focusing quality criterion (for example based on the pixel in the center of the field), or only few focusing quality criteria (for example based on only some pixels distributed across the field).

For example, the ultrasound attenuation property may be estimated based on an amplitude variation across the depth direction of the spatial region represented by the second subset. In case the area 50a comprises a plurality of second subsets/regions 40a which have a same depth level in the medium, the method may further comprise estimating an ultrasound attenuation property across of the selected regions and averaging the attenuation properties.

In case the area 50a comprises a plurality of second subsets/regions 40a which have different depth levels in the medium, the method may further comprise estimating an ultrasound attenuation property for the entirety of selected regions (i.e. across the entire depth direction of the selected regions 40a). In this way, an attenuation property can be determined based on an area which extends further in the depth direction. The determination can thus be more reliable and/or more precise.

The pattern resolution (i.e. the grid size resolution) may be adaptable/adapted as a function of the determined homogeneity properties. For example, the pattern may have a predefined initial resolution (mesh size), wherein in case none of the regions of the pattern is selected, the resolution may be increased (i.e. the mesh size of the grid decreased). In this way, it becomes possible to start with a lower resolution which requires less computational power, and to increase the resolution, only when and/or where it is appropriate.

Fig. 6 shows an example of evaluating ultrasound data based on phase properties for the purpose of ultrasound attenuation estimation. The evaluation technique may be implemented in the system of Fig. 1 and/or according to the method of Fig. 3a and/or 3b. A plurality 20 of transducer elements 21 are displayed on the top. Spatio-temporal signal data 50 are indicated below. The spatio-temporal signal data comprise a plurality of signals respectively received by the plurality of transducer element elements 21 during a period of time. Specific subsets 51 of each signal are schematically illustrated. The subsets 51 are located along a dotted line 60. The dotted line 60 may be theoretical line and/or an imaginary line and/or may be understood as an illustration, for example as an illustration of operation (c) of determining a subset of spatio-temporal signal data as a function of a predefined spatial region 40 in the medium 10. The shape of the dotted line is related to an actual wave propagation speed in the medium. The shape of the dotted line 60 may be related to the location of a spatial region in the medium reflecting a transmitted ultrasonic pulse. The dotted line 60 may be precomputed. In other words, the sections of the signals covered by the dotted line may be associated with the spatial region. Based on said sections, a pixel of beamformed image data may be calculated in an optional beamforming operation.

The method described in context of fig. 3a may comprise an operation (not shown in fig. 3a) of determining a first subset of the spatio-temporal signal data as a function of a predefined spatial region in the medium. Said operation may either be part of the beamforming process of operation (b) or be an additional operation prior to operations (b) and (c) and after operation (a). This operation of determining the first subset may be understood as corresponding to drawing/determining/building the dotted line 60 in Fig. 6. Phase properties of the respective individual signals along the dotted line may be compared to each other in the operation (c) of determining a focusing quality criterion.

For example, spatial temporal signal data, which may be RF data or pre-beamformed data, may comprise n signals from n transducer elements 21. Spatial temporal signal data 50 lying below (i.e. covered by) the dotted line 60 may correspond to sections of a plurality of oscillating signals, for example of the spatio-temporal signal data. Spatial temporal signal data 50 lying below the dotted line 60 may correspond to a subset of the spatio-temporal signal data 50 that may be associated with a predefined spatial region 40 in the medium 10. The subset may comprise one instance in time of a corresponding signal, for example of a signal corresponding to a respective transducer, and/or of a plurality of instances in time of a signal corresponding to a respective transducer. The focusing quality criterion may be determined in operation (c) based on phase properties of the first subset. For example, a sign proportion may be used as the focusing quality criterion. For example, out of 100 signals corresponding to 100 respective transducers, 70 signals may exhibit a positive sign and 30 may exhibit a negative sign. A corresponding sign proportion of for example 70% may then be determined. The second subset of the present disclosure (for example a beamformed image pixel determined based on the first subset) may then be evaluated based on the determined figure of merit, for example based on the sign proportion of 70%.

It is possible that the method not only takes into account the phase properties (for example the sign "+" or "-" of the signal) but also its amplitude, in order to determine the focusing quality criterion. In case the amplitude is taken into account, the determined focusing quality criterion can be more precise, as the energy level of the signals at the dotted line (which represents the first subset) is considered. Accordingly, also the evaluation result (i.e. the figure of merit) can be more precise and/or more reliable.

Moreover, taking the amplitude into account may be advantageous, in order to compare the resulting figures of merit of different second subsets (i.e. different spatial regions), for example to check whether they are homogeneous or not.

However, in case the amplitude is not taken into account, the determined method can be computationally less expensive (i.e. faster) and at the same time more robust, as in this case it is only necessary to determine whether an oscillating signal is positive ("+") or negative ("-") at an given instance of time (as marked by the dotted line 60) signal. Small phase deviations usually do not influence the result, as the signal is half the period positive and half the period negative. Accordingly, both alternatives (i.e. taking the amplitude into account or not) can be advantageous.

The phase properties may hence also provide information of a level of noise in the first subset. For example, in case the phase properties are not consistent (for example the signs are 50% positive and 50% negative), it may be determined that the first subset is noisy.

Throughout the description, including the claims, the term "comprising a" should be understood as being synonymous with "comprising at least one" unless otherwise stated. In addition, any range set forth in the description, including the claims should be understood as including its end value(s) unless otherwise stated. Specific values for described elements should be understood to be within accepted manufacturing or industry tolerances known to one of skill in the art, and any use of the terms "substantially" and/or "approximately" and/or "generally" should be understood to mean falling within such accepted tolerances.

The terms "record" and "receive" may be used synonymously throughout this disclosure unless denoted differently.

Although the present disclosure herein has been described with reference to particular examples, it is to be understood that these examples are merely illustrative of the principles and applications of the present disclosure.

It is intended that the specification and examples be considered as exemplary only, with a true scope of the disclosure being indicated by the following claims.

A reference herein to a patent document or any other matter identified as prior art, is not to be taken as an admission that the document or other matter was known or that the information it contains was part of the common general knowledge as at the priority date of any of the claims.

**Claims**

1. A method of evaluating ultrasound data for the purpose of ultrasound attenuation estimation in a medium, the medium being associated with ultrasound spatio-temporal signal data received from the medium in response to at least one ultrasound plane and/or diverging wave emitted into the medium,
   wherein the method comprises:

   beamforming a first subset of ultrasound spatio-temporal signal data to obtain a second subset of beamformed ultrasound data,
   determining a focusing quality criterion from the first subset, and
   evaluating the second subset as a function of the focusing quality criterion of the first subset.

2. The method according to claim 1, wherein

   the first subset is selected in a predefined beamforming process as a function of a first predefined spatial region in the medium, and
   the second subset comprises image data of the first predefined spatial region.

3. The method according to claim 1 or 2, wherein
   the focusing quality criterion comprises at least one of:

   a coherence property of the first subset, and
   phase properties of the first subset.

4. The method according to any. one of the preceding claims, further comprising: determining a signal-to-noise ratio parameter from the first subset, and evaluating the second subset as a function of the signal-to-noise ratio parameter of the first subset.

5. The method according to any one of the preceding claims, wherein

   the focusing quality criterion comprises a B-mode ratio, and/or
   a coherence ratio determined by:

      normalizing the beamforming process by an incoherent energy arising from the first predefined spatial region, and/or
      determining a coherent sum and a non-coherent sum of the first subset and setting them in a ratio.

6. The method according to the preceding claim, wherein
   the phase properties comprise at least one of:

      • signs,
      • phases,
      • a sign proportion,
      • phase intervals,
      • phase evolution states, and
      • proportions of phase properties.

7. The method according to any one of the preceding claims 3 to 6, wherein the phase properties are determined by using a predefined function G, the function G being at least one of:

   a function $G(x)$ that is symmetric about the axis $x = 50\%$, configured to maximize at $x=50\%$ and minimize at x=0% and x=100% or to minimize at x=50% and maximize at x=0% and x=100%,
a bell-shaped function,
a normal distribution function,
a Shannon entropy function.

8. The method according to any one of the preceding claims, wherein evaluating the second subset comprises comparing the focusing quality criterion of the respective first subset with a predefined range.

9. The method according to the preceding claim, wherein

the second subset is evaluated to be useable for the purpose of ultrasound attenuation estimation in a medium, in case the focusing quality criterion of the respective first subset is within the predefined range, and/or
the second subset is evaluated to be not useable for the purpose of ultrasound attenuation estimation in a medium, in case the focusing quality criterion of the respective first subset is outside the predefined range.

10. The method according to any one of the preceding claims, wherein beamforming a first subset comprises:

- beamforming a plurality of first subsets of ultrasound spatio-temporal signal data to obtain a respective plurality of second subset of beamformed ultrasound data, wherein the plurality of first subsets is associated with different spatial regions in the medium, and/or
- determining a focusing quality criterion comprises:
determining for each of a plurality of first subsets a focusing quality criterion.

11. The method according to any one of the preceding claims, wherein evaluating the second subset comprises:

evaluating a plurality of second subsets as a function of the focusing quality criteria of the respective first subsets, and optionally
generating a focusing quality map based on the evaluated plurality of second subsets.

12. The method according to any one of the preceding claims, further comprising:
evaluating an area in the focusing quality map comprising a plurality of adjacent second subsets as a function of a difference between the focusing quality criteria of the respective first subsets.

13. The method according to any one of the preceding claims, wherein determining a focusing quality criterion comprises:
determining a focusing quality criterion from the first subset and additionally from at least one further first subset associated with a neighboring spatial region with respect to the first predefined spatial region.

14. The method according to any one of the preceding claims, further comprising: determining a speckle statistic of the second set, wherein the second set optionally comprises IQ beamformed ultrasound data.

15. A method of estimating an ultrasound attenuation property in a medium, comprising:

a method according to any of one of the preceding claims,
selecting at least one evaluated second subset as a function of the respective focusing quality criterion, and
estimating the ultrasound attenuation property by applying a predefined attenuation estimation method to the selected second subset.

16. A method of estimating an ultrasound backscattering coefficient in a medium, comprising:

a method according to any of one of the preceding claims,
selecting at least one evaluated second subset as a function of the respective focusing quality criterion and/or as a function of the respective ultrasound attenuation property, and
estimating the ultrasound backscattering coefficient by applying a predefined backscattering estimation method to the selected second subset.

17. A computer program comprising computer-readable instructions which when executed by a data processing system cause the data processing system to carry out the method according to any one of preceding method claims.

**18.** A system for evaluating ultrasound data for the purpose of ultrasound attenuation estimation in a medium, the medium being associated with ultrasound spatio-temporal signal data received from the medium in response to at least one ultrasound plane and/or diverging wave emitted into the medium, wherein the system comprises a processing unit configured to:

beamform a first subset of ultrasound spatio-temporal signal data to obtain a second subset of beamformed ultrasound data,
determine a focusing quality criterion from the first subset, and evaluate the second subset as a function of the focusing quality criterion of the first subset.

**Fig. 1**

**Fig. 2**

f1

(a): provide ultrasound spatio-temporal signal data of the medium

(a1) (optional): sending pulse (plane and/or diverging wave)

(a2) (optional): Receive in response the plurality of signals from the medium

ultrasound spatio-temporal signal data of the medium

(b): beamforming a first subset of ultrasound spatio-temporal signal data

(b1) (optional): beamforming a plurality of first subsets of ultrasound spatio-temporal signal data

(c): determining a focusing quality criterion from the first subset

(c1) (optional): determining for each of a plurality of first subsets a focusing quality criterion

second subset(s) of beamformed ultrasound data

focusing quality criterion

(d): evaluating the second subset as a function of the focusing quality criterion of the first subset

(d1) (optional): evaluating a plurality of second subsets as a function of the focusing quality criteria of the respective first subsets

evaluation result (optionally with a speckle statistic)

# Fig. 3a

f2

evaluation results for
a plurality of second subsets

(e): generating a focusing quality map

(f): selecting at least one evaluated second
subset

(g): estimating the ultrasound attenuation
property

# Fig. 3b

**Fig. 4a**

**Fig. 4b**

**Fig. 5**

**Fig. 6**

Europäisches
Patentamt

European
Patent Office

Office européen
des brevets

## EUROPEAN SEARCH REPORT

Application Number

EP 22 31 5178

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/018968 A1 (KONINKLIJKE PHILIPS NV [NL]) 4 February 2021 (2021-02-04) * Pages 5-10,13-15,18 Equation 10; figures 1,2 * | 1-18 | INV. G01S7/52 A61B8/08 |
| A | US 2017/105705 A1 (YOON HEE-CHUL [KR] ET AL) 20 April 2017 (2017-04-20) * paragraphs [0039] - [0046]; figure 1 * | 4 | |
| A | US 2020/390421 A1 (AUDIÈRE STÉPHANE [FR] ET AL) 17 December 2020 (2020-12-17) * paragraphs [0060] - [0076]; claims 1-5 * | 1-18 | |

TECHNICAL FIELDS
SEARCHED        (IPC)

G01S
A61B

The present search report has been drawn up for all claims

| Place of search | Date of completion of the search | Examiner |
|---|---|---|
| Munich | 19 January 2023 | Theißing, Nikolaus |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
..................................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

**EP 22 31 5178**

This annex lists the patent family members relating to the patent documents cited in the above-mentioned European search report.
The members are as contained in the European Patent Office EDP file on
The European Patent Office is in no way liable for these particulars which are merely given for the purpose of information.

**19-01-2023**

| Patent document cited in search report | | Publication date | Patent family member(s) | | Publication date |
|---|---|---|---|---|---|
| WO 2021018968 | A1 | 04-02-2021 | CN | 114207431 A | 18-03-2022 |
| | | | EP | 4004592 A1 | 01-06-2022 |
| | | | JP | 2022542941 A | 07-10-2022 |
| | | | US | 2022280138 A1 | 08-09-2022 |
| | | | WO | 2021018968 A1 | 04-02-2021 |
| US 2017105705 | A1 | 20-04-2017 | CA | 2943666 A1 | 01-10-2015 |
| | | | CN | 106464868 A | 22-02-2017 |
| | | | EP | 3108654 A1 | 28-12-2016 |
| | | | JP | 6280235 B2 | 14-02-2018 |
| | | | JP | 2017512570 A | 25-05-2017 |
| | | | KR | 20150111698 A | 06-10-2015 |
| | | | US | 2017105705 A1 | 20-04-2017 |
| | | | WO | 2015147471 A1 | 01-10-2015 |
| US 2020390421 | A1 | 17-12-2020 | NONE | | |

EPO FORM P0459

For more details about this annex : see Official Journal of the European Patent Office, No. 12/82

## REFERENCES CITED IN THE DESCRIPTION

**Patent documents cited in the description**

- EP 3936891 A1

- US 10932750 B2

**Non-patent literature cited in the description**

- **LIN XIN YAO ; JAMES A. ZAGZEBSKI ; ERNEST L. MADSEN.** Backscatter coefficient measurements using a reference phantom to extract depth-dependent instrumentation factors. *Ultrasonic Imaging,* 1990, vol. 12 (1), ISSN 0161-7346, 58-70

- **MALLART, RAOUL ; MATHIAS FINK.** Adaptive focusing in scattering media through sound-speed inhomogeneities: The van Cittert Zernike approach and focusing criterion. *Journal of the Acoustical Society of America,* 1994, vol. 96, 3721-3732